(19) Europäisches Patentamt
European Patent Office
Office européen des brevets

(11) Numéro de publication: **0 082 059**
**A1**

(12) **DEMANDE DE BREVET EUROPEEN**

(21) Numéro de dépôt: **82402223.0**

(22) Date de dépôt: **06.12.82**

(51) Int. Cl.³: **C 07 D 317/58,** C 07 C 131/00, A 61 K 31/15

(30) Priorité: **11.12.81 FR 8123148**

(43) Date de publication de la demande: **22.06.83 Bulletin 83/25**

(84) Etats contractants désignés: **CH DE FR GB IT LI**

(71) Demandeur: **UNIVABLOT, 21 bis rue Jonquoy, F-75014 Paris (FR)**

(72) Inventeur: **Astoin, Jacques Noel, 5 rue Basse des Carmes, F-75005 Paris (FR)**
Inventeur: **Lepage, Francis, 13 rue du Général de Larminat, F-94000 Creteil (FR)**
Inventeur: **Fromantin, Jean-Pierre Marie Joseph, 50 avenue Villeneuve l'Etang, F-78000 Versailles (FR)**

(74) Mandataire: **Lepeudry-Gautherat, Thérèse et al, CABINET ARMENGAUD JEUNE CASANOVA et LEPEUDRY 23 boulevard de Strasbourg, F-75010 Paris (FR)**

(54) **Ethers d'oximes alpha-bêta insaturés, leur procédé de préparation et leur utilisation comme médicament.**

(57) L'invention concerne des composés d'éthers d'oximes répondant à la formule générale (I) ci-après

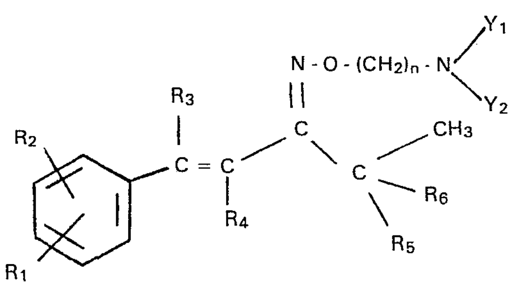

L'invention concerne également des procédés de préparation des éthers d'oximes de formule générale (I).

Les composés selon l'invention sont utilisables comme médicaments notamment pour leur action spasmolytique et anti-histaminique.

EP 0 082 059 A1

La présente invention concerne des éthers d'oximes α-β insaturés, leur procédé de préparation et leur utilisation comme médicament.

Les composés selon l'invention répondent à la formule générale (I) :

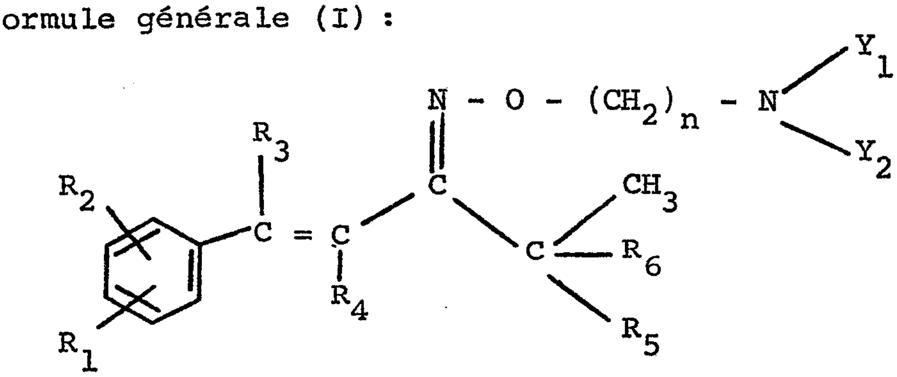

(I)

dans laquelle

$R_1$ et $R_2$ identiques ou différents représentent un atome d'hydrogène, un atome d'halogène, un radical alkyle de 1 à 4 atomes de carbone, ou bien $R_1$ et $R_2$ pris ensemble représentent un groupe méthylènedioxy,

$R_3$ représente un atome d'hydrogène ou un radical phényle éventuellement substitué par un radical alkyle en $C_1$-$C_4$ ou par un atome d'halogène,

$R_4$ représente un atome d'hydrogène,

$R_5$ représente un groupe méthyle ou pris en combinaison avec le radical $R_4$ représente une chaîne alkylène en $C_3$,

$R_6$ représente un atome d'hydrogène ou le radical méthyle,

$Y_1$ et $Y_2$ identiques ou différents représentent un radical alkyle linéaire ou ramifié ayant de 1 à 4 atomes de carbone, ou bien $Y_1$ et $Y_2$ représentent conjointement une chaîne alkylène en $C_4$-$C_7$ pouvant également éventuellement contenir un hétéroatome d'oxygène ou d'azote,

n est un nombre entier compris entre 1 et 4, ainsi que les sels, les dérivés d'ammonium quaternaire, les stéréoisomères possibles et leur mélange, desdits éthers oximes. De préférence n est choisi entre 2 et 3.

Les composés de formule (I) peuvent être préparés par les procédés suivants.

Selon un premier procédé, on fait réagir une cétone de formule générale (II)

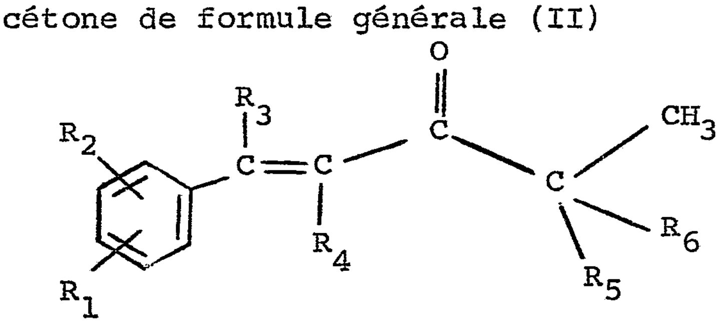

(II)

avec un dérivé d'hydroxylamine de formule générale (III)

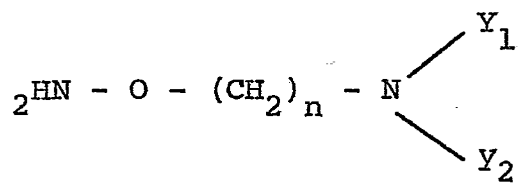

(III)

dans lesquelles $R_1$, $R_2$, $R_3$, $R_4$, $R_5$, $R_6$, $Y_1$, $Y_2$ et n ont la signification ci-dessus en présence de préférence d'un solvant inerte vis-à-vis de la réaction, par exemple l'éthanol ou pyridine.

Selon un deuxième procédé, on fait réagir une oxime de formule générale (IV)

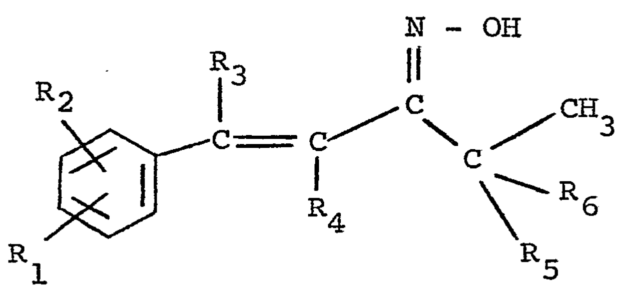

(IV)

avec un dérivé halogénoalkylaminé de formule générale (V)

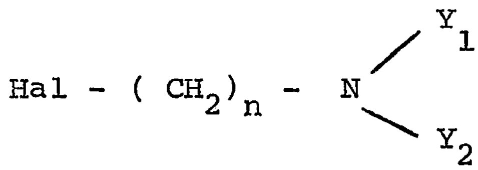

(V)

dans lesquelles $R_1$, $R_2$, $R_3$, $R_4$, $R_5$, $R_6$, $Y_1$, $Y_2$ et n ont la signification ci-dessus. La réaction a lieu dans un solvant inerte tel que par exemple le diméthylformamide en présence d'un agent de condensation basique tel que des hydrures de métaux alcalins par exemple l'hydrure de sodium.

Les cétones de formule générale (II) peuvent être préparées par divers procédés.

Les cétones pour lesquelles $R_3 = R_4 = H$ sont pour la plupart connues et peuvent être obtenues par condensation en milieu aqueux ou alcoolique en quantités équimoléculaires des aldéhydes de formule générale (VI) :

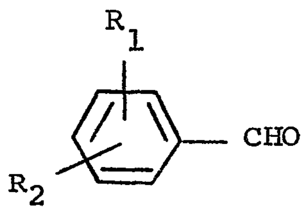

(VI)

avec une cétone de formule générale (VII) :

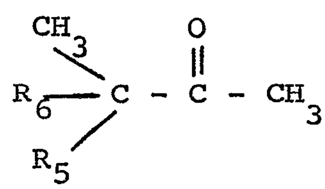

(VII)

dans lesquelles $R_1$, $R_2$, $R_5$ et $R_6$ ont la signification ci-dessus.

Les autres cétones de formule générale (II) dans laquelle $R_3$ est différent de H peuvent être préparées par action d'un organomagnésien de formule (VIII) :

$R_3MgHal$ (VIII)

sur une cétone de formule générale (IX) :

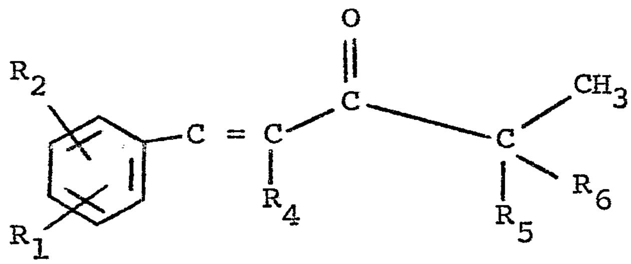

(IX)

dans lesquelles $R_1,R_2,R_3,R_4,R_5$ ont la signification ci-dessus.

On halogène la cétone saturée correspondante puis on élimine une molécule d'hydracide afin d'obtenir la cétone insaturée de formule générale (II). Cette méthode donne un mélange d'isomères cis-trans que l'on peut éventuellement séparer.

Les oximes de formule générale (IV) peuvent être préparées en faisant réagir en quantité équimolaire du chlorhydrate d'hydroxylamine sur une cétone de formule générale (II).

Les composés de formule (I) peuvent être transformés de manière connue en leurs sels d'addition avec un acide ou en leurs sels d'ammonium quaternaire.

On prépare les sels d'addition d'acide avec des acides physiologiquement acceptables tels que l'acide citrique, l'acide fumarique, l'acide acétique etc..

Pour préparer les sels d'ammonium quaternaire l'on fait réagir des composés de formule générale (I) avec des composés propres à se transformer en dérivés quaternaires tels que par exemple un halogénure d'alkyle ou un ester d'acide méthane-sulfonique.

Les composés selon l'invention ainsi que leurs sels d'addition physiologiquement acceptables sont utilisables comme médicaments notamment pour leur action spasmolytique et anti-histaminique, par exemple pour le traitement des maladies cardiovasculaires.

Les composés selon l'invention peuvent être présentés en association avec un excipient pharmaceutiquement compatible pour l'administration par voie orale, par exemple sous forme de comprimés, dragées ou gélules et pour

l'administration par voie parentérale sous forme de solutions injectables pour les composés solubles ou pour l'administration endorectale sous forme de suppositoires.

La posologie quotidienne est de l'ordre de 80 à 300 mg par voie orale et d'environ 8 à 60 mg par voie injectable.

Pour mettre en évidence l'effet spasmolytique, on utilise la méthode de MAGNUS (Versuche am überbenden Dünndarm von Saugetieren - Arch. Gas. Physiol 1904, 102-123). On mesure l'activité anti-spasmodique sur le spasme provoqué par l'acétylcholine ou le chlorure de baryum, au niveau du duodénum isolé du rat.

Un rat est sacrifié, après ouverture de l'abdomen la portion duodénale est prélevée et sa lumière est soigneusement lavée avec une solution de Tyrode à 37°C. Le fragment d'intestin est fixé dans un bain à organe isolé à 37°C et oxygéné. On recherche la dose d'acétylcholine ou de chlorure de baryum exprimée en mole par litre qui additionnée au bain provoque une réponse contractile de l'organe fournissant un tracé sur papier de l'enregistreur. Après lavage de l'organe on recherche la dose de produit selon l'invention qui additionnée au bain durant une minute avant la dose d'acétylcholine ou de chlorure de baryum définie, réduit le spasme de moitié. On opère de la même façon avec un produit de référence, l'atropine pour l'acétylcholine et la papavérine pour le chlorure de baryum. On exprime ainsi pour le produit à étudier et le produit de référence, la dose efficace 50.

On met en évidence l'action anti-histaminique de la même façon en utilisant l'ilion isolé de cobaye. La méthode est rigoureusement la même, seul l'agent contracturant change. Il est pour cet essai l'histamine et le produit de référence est la prométhazine. Les résultats de ces essais sont indiqués dans le tableau I ci-après.

D'autres avantages et caractéristiques de l'invention apparaîtront à la lecture des exemples suivants donnés à titre illustratif nullement limitatif, préparés

à partir des cétones de formule (II) comme indiqué ci-dessus. La plupart de ces cétones sont connues et décrites dans la littérature, sauf la (méthylènedioxy-3,4 phényl)-1 phényl-1 diméthyl-4,4 pentène-1 one-3, dont la préparation est décrite ci-dessous :

On ajoute goutte à goutte une suspension éthérée de 0,17 mole de (méthylène-dioxy-3,4 phényl)-1 diméthyl-4,4 pentène-1 one-3, dont la synthèse est décrite dans J. Chem. Soc, 1910, 97, 1753, dans une solution de phényl-bromomagnésien préparée à partir de 0,17 mole de bromobenzène et de 0,17 atome gramme de magnésien dans 150 ml d'éther. On porte au reflux 1/2 heure. On ajoute goutte à goutte 0,22 mole de bromure et on verse ensuite une solution de HCl 10% en volume. On extrait, on sèche, on concentre et on recristallise la bromacétone intermédiaire (F = 168°C, rendement 90%). On reprend ce dérivé bromé par 500 ml de solution hydroalcoolique potassique (15 g de potasse en pastille (85%) dans 500 ml d'éthanol à 90°); on porte au reflux pendant 1 heure. On concentre l'éthanol, on reprend par de l'eau et on extrait à l'acétate d'éthyle et on distille à 215°C/2 mmHg. On obtient avec un rendement de 87% un mélange d'isomère cis-trans dans des proportions respectives 15 : 85.

Préparation des éthers d'oximes.

EXEMPLE 1 :

Ether O-(diéthylaminoéthylique) de l'oxime de la (méthylène-dioxy-3,4 phényl)-1 diméthyl-4,4 pentène-1 one-3.

a) A partir de la cétone:(méthylène-dioxy-3,4 phényl)-1 diméthyl-4,4 pentène-1 one-3 dont la préparation est décrite dans (J. Chem. Soc., 1910, 97, 1753).

On porte au reflux pendant 3 heures, 0,1 mole de cétone, 0,1 mole de chlorhydrate de diéthylamino-

éthoxylamine en solution dans 150 ml d'éthanol anhydre en présence de 75 ml de pyridine. On concentre, on alcalinise et on extrait au benzène. On obtient le produit désiré avec un rendement de 65%.

b) A partir de l'oxime: (méthylène-dioxy-3,4 phényl)-1 diméthyl-4,4 pentène-1 one-3 oxime :on ajoute lentement 1,4 g d'hydrure de sodium à 50% dans l'huile à une solution de 0,03 mole d'oxime dans du diméthylformamide. On ajoute ensuite 0,03 mole de chloro-2 éthyldiéthylamine. Après 6 heures d'agitation à 70°C, on verse l'eau extraite à l'éther. On sèche et on concentre, on obtient le produit désiré avec un rendement de 51%.

EXEMPLES 2 à 21 :

En reprenant le mode opératoire de l'exemple 1 et en utilisant comme produit de départ la cétone ou l'oxime correspondante on a réalisé la synthèse des produits suivants :

EXEMPLE 2 :

Ether O-diméthylaminoéthylique de l'oxime de la (méthylènedioxy-3,4 phényl)-1 diméthyl-4,4 pentène-1 one-3.

EXEMPLE 3 :

Ether O-(diisopropylaminoéthylique) de l'oxime de la (méthylènedioxy-3,4 phényl)-1 diméthyl-4,4 pentène-1 one-3.

EXEMPLE 4 :

Ether O-(morpholinoéthylique) de l'oxime de la (méthylènedioxy-3,4 phényl)-1 diméthyl-4,4 pentène-1 one-3.

EXEMPLE 5 :

Ether O-(pyrrolidinoéthylique) de l'oxime de la (méthylène dioxy-3,4 phényl)-1 diméthyl-4,4 pentène-1 one-3.

EXEMPLE 6 :

Ether O-(diméthylaminopropylique) de l'oxime de la (méthylène dioxy-3,4 phényl)-1 diméthyl-4,4

pentène-1 one-3.

EXEMPLE 7 :

Iodure d'éther O-(diéthylméthylammonio-éthylique) de l'oxime de la (méthylènedioxy-3,4 phényl)-1 phényl-1 diméthyl-4,4 pentène-1 one-3.

EXEMPLE 8 :

Ether O-(diméthylaminopropylique) de l'oxime de la (méthylènedioxy-3,4 phényl)-1 phényl-1 diméthyl-4,4 pentène-1 one-3.

EXEMPLE 9 :

Ether O-(diéthylaminoéthylique) de l'oxime de la (méthylènedioxy-3,4 benzylidène)-2 diméthyl-6,6 cyclohexanone-1.

EXEMPLE 10 :

Ether O-(diméthylaminopropylique) de l'oxime de la (méthylènedioxy-3,4 benzylidène)-2 diméthyl-6,6 cyclohexanone-1.

EXEMPLE 11 :

Iodure d'éther O-( triméthylammoniopropylique) de l'oxime de la (méthylènedioxy-3,4 benzylidène)-2 diméthyl-6,6 cyclohexanone-1 .

EXEMPLE 12 :

Ether O-(diéthylaminopropylique) de l'oxime de la (méthylènedioxy-3,4 benzylidène)-2 méthyl-6 cyclohexanone-1.

EXEMPLE 13 :

Ether O-(morpholinoéthylique) de l'oxime de la (méthylènedioxy-3,4 benzylidène)-2 méthyl-6 cyclohexanone-1.

EXEMPLE 14 :

Ether O-(diéthylaminoéthylique) de l'oxime de la (p-chloro-phényl)-1 diméthyl-4,4 pentène-1 one-3.

EXEMPLE 15 :

Ether O-(diméthylaminopropylique) de

l'oxime de la (p-chlorophényl)-1 diméthyl-4,4 pentène-1 one-3.

EXEMPLE 16 :

Ether O-(pipéridinoéthylique) de l'oxime de la (p-chlorophényl)-1 diméthyl-4,4 pentène-1 one-3.

EXEMPLE 17 :

Ether O-(diéthylaminoéthylique) de l'oxime de la (dichloro-2,4 phényl)-1 diméthyl-4,4 pentène-1 one-3.

EXEMPLE 18 :

Ether O-(diéthylaminoéthylique) de l'oxime de la (dichloro-2,4 benzylidène)-2 méthyl-6 cyclohexanone-1 .

EXEMPLE 19 :

Ether O-(diéthylaminoéthylique) de l'oxime de la phényl-1 diméthyl-4,4 pentène-1 one-3.

EXEMPLE 20 :

Ether O-(diméthylaminopropylique) de l'oxime de la (dichloro-2,4 benzylidène)-2 diméthyl-6,6 cyclohexanone-1.

EXEMPLE 21 :

Ether O-(diéthylaminoéthylique) de l'oxime de la (dichloro-3,4 benzylidène)-2 méthyl-6 cyclohexanone-1.

Les formules des exemples 1 à 21, les constantes physiques et les résultats des essais pharmacologiques sont consignés dans le tableau I ci-après.

Des résultats de ces essais, il apparaît que les composés présentent une activité spasmolytique intéressante.

TABLEAU I

| Ex. | FORMULE | CONSTANTE Pt FUSION F °C | ACETYLCHOLINE | | CHLORURE DE BARYUM | | HISTAMINE | |
|---|---|---|---|---|---|---|---|---|
| | | | $C_1$ | $DE_{50}$ | $C_2$ | $DE_{50}$ | $C_3$ | $DE_{50}$ |
| 1 | 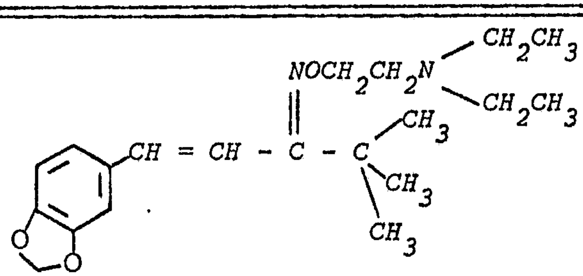 | Chlorhy. 126 | $10^{-7}$<br>$10^{-8}$ | 49<br>38 | $7,5\ 10^{-7}$ | 35 | $10^{-6}$ | 50 |
| 2 | 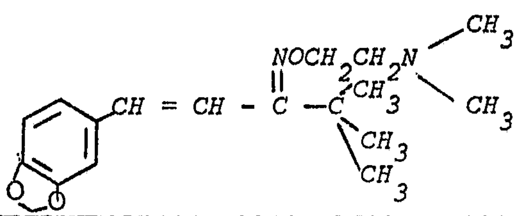 | Chlorhy. 185 | $10^{-6}$ | 51 | | | $10^{-6}$ | 50 |
| 3 | 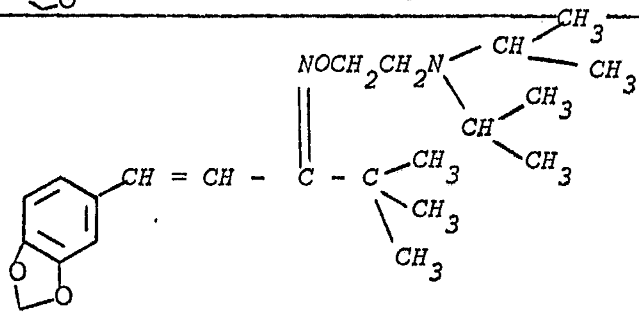 | Chlorhy. 118 | $10^{-6}$ | 57 | | | $10^{-6}$ | 13 |

TABLEAU I (suite)

| Ex. | FORMULE | CONSTANTE Pt DE FUSION F°C | ACETYLCHOLINE $C_1$ | ACETYLCHOLINE $DE_{50}$ | CHLORURE DE BARUYM $C_2$ | CHLORURE DE BARUYM $DE_{50}$ | HISTAMINE $C_3$ | HISTAMINE $DE_{50}$ |
|---|---|---|---|---|---|---|---|---|
| 4 | 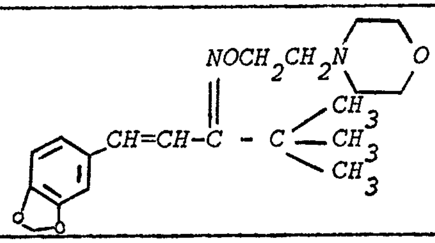 | Chlorhy. 163 | $2,5.10^{-6}$ | 40 | | | | |
| 5 | 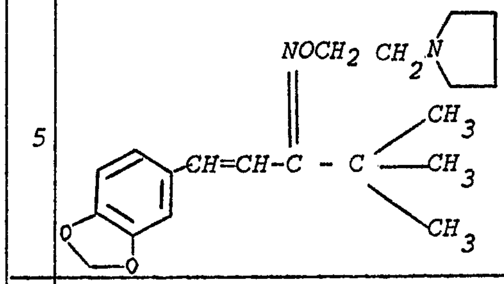 | Chlorhy. 122 | $10^{-6}$ | 67 | | | $10^{-6}$ | 40 |
| 6 | 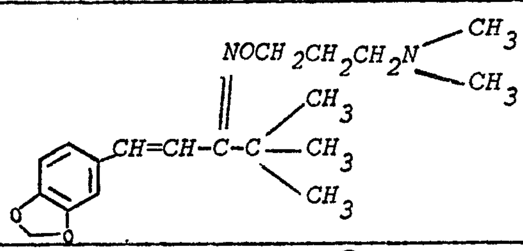 | Chlorhy. 138 | $5.10^{-6}$ | 56 | | | $10^{-6}$ | 11 |
| 7 | 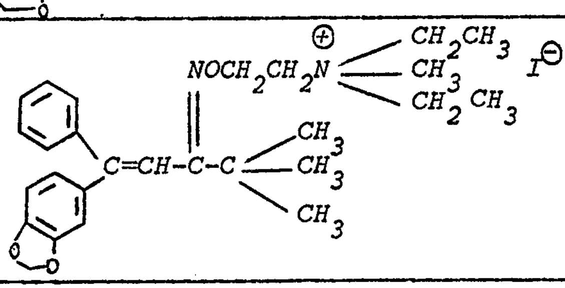 | 166 | $10^{-6}$ | 49 | | | $10^{-6}$ | 52 |

TABLEAU I (suite)

| Ex. | FORMULE | CONSTANTES Pt DE FUSION F°C | ACETYLCHOLINE | | CHLORURE DE BARYUM | | HISTAMINE | |
|---|---|---|---|---|---|---|---|---|
| | | | $C_1$ | $DE_{50}$ | $C_2$ | $DE_{50}$ | $C_3$ | $DE_{50}$ |
| 8 | 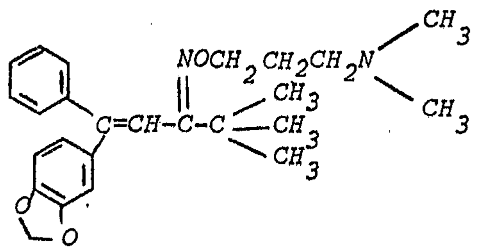 | Chlorhy. 90 | $10^{-6}$ | 44 | | | $10^{-6}$ | 43 |
| 9 | 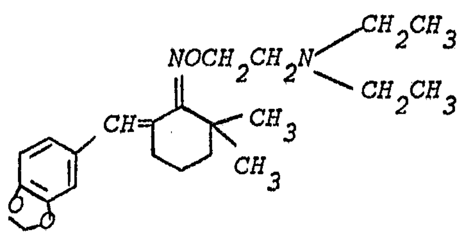 | Chlorhy. 163 | $5.10^{-7}$ | 60 | $2,5.10^{-7}$ | 59 | $5.10^{-6}$ | 48 |
| 10 | 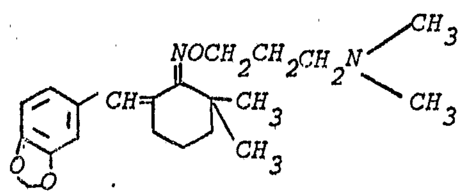 | Chlorhy. 145 | $10^{-6}$ | 62 | | | $5.10^{-7}$ | 44 |
| 11 | 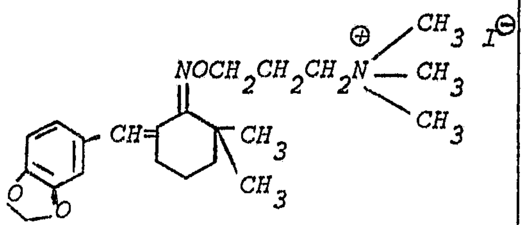 | 155 | $10^{-6}$ | 41 | | | $10^{-5}$ | 35 |

T A B L E A U I (suite)

| Ex. | FORMULE | CONSTANTES Pt DE FUSION F°C | ACETYLCHOLINE | | CHLORURE DE BARYUM | | HISTAMINE | |
|---|---|---|---|---|---|---|---|---|
| | | | $C_1$ | $DE_{50}$ | $C_2$ | $DE_{50}$ | $C_3$ | $DE_{50}$ |
| 12 | 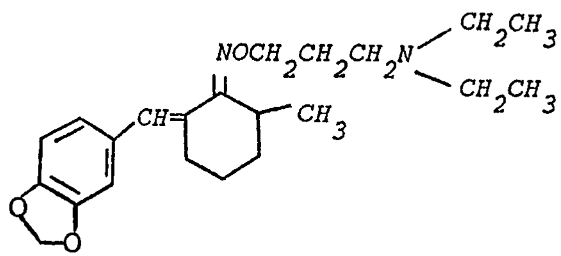 | Chlorhy. 140 | $2,5.10^{-7}$ | 58 | $10^{-6}$ | 49 | $10^{-6}$ | 34 |
| 13 | 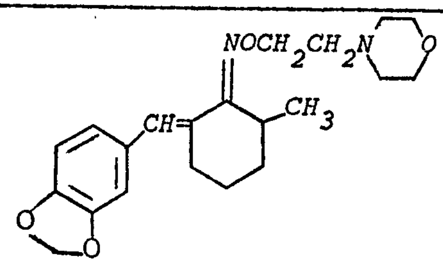 | Chlorhy. 132 | $510^{-6}$ | 56 | | | | |
| 14 | 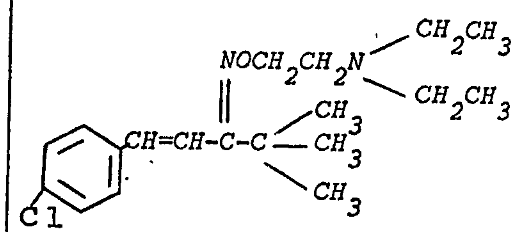 | Citrate env.120 | $10^{-6}$ | 40 | | | $10^{-5}$ | 88 |
| 15 | 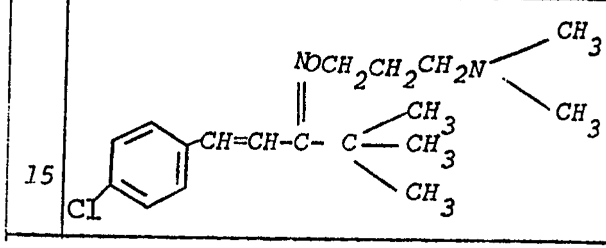 | Citrate env.108 | $10^{-6}$ | 33 | | | $10^{-6}$ | 40 |

T A B L E A U   I   (suite)

| Ex. | FORMULE | CONSTANTES Pt DE FUSION F°C | ACETYLCHOLINE | | CHLORURE DE BARYUM | | HISTAMINE | |
|---|---|---|---|---|---|---|---|---|
| | | | $C_1$ | $DE_{50}$ | $C_2$ | $DE_{50}$ | $C_3$ | $DE_{50}$ |
| 16 | 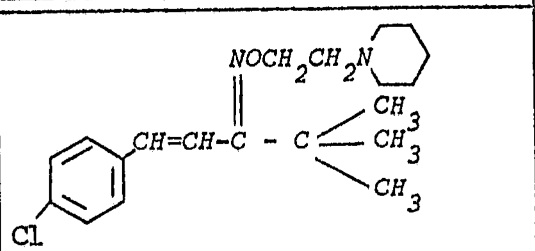 | Citrate 132 | $10^{-6}$ | 48 | | | $10^{-5}$ | 88 |
| 17 | 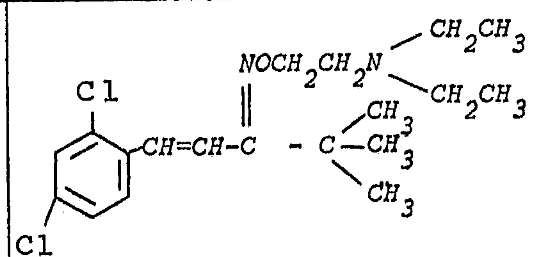 | Citrate env.86 | $10^{-5}$ | 27 | $10^{-5}$ | 60 | $10^{-5}$ | 57 |
| 18 | 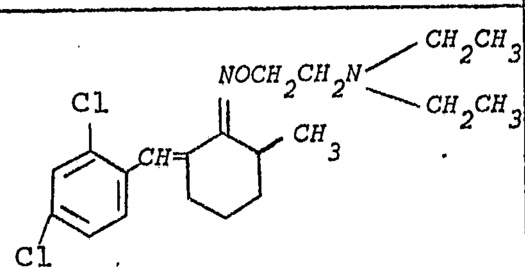 | Chlorhy. 131 | $10^{-6}$ | 50 | | | $10^{-5}$ | 52 |
| 19 | 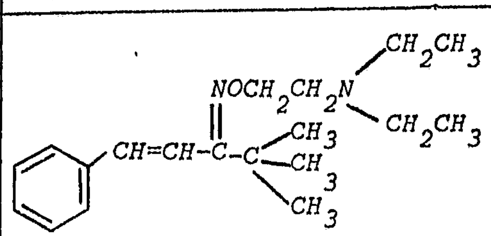 | Chlorhy. 110 | $10^{-6}$ | 37 | | | $10^{-6}$ | 42 |

*T A B L E A U I (suite et fin)*

| Ex. | FORMULE | CONSTANTES Pt DE FUSION F°C | ACETYLCHOLINE | | CHLORURE DE BARYUM | | HISTAMINE | |
|---|---|---|---|---|---|---|---|---|
| | | | $C_1$ | $DE_{50}$ | $C_2$ | $DE_{50}$ | $C_3$ | $DE_{50}$ |
| 20 | 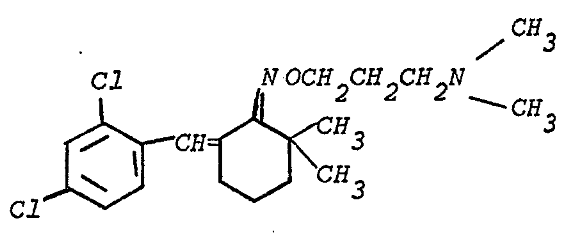 | Chlorhy. 128 | $10^{-6}$ | 40 | $10^{-5}$ | 82 | $10^{-5}$ | 82 |
| 21 | 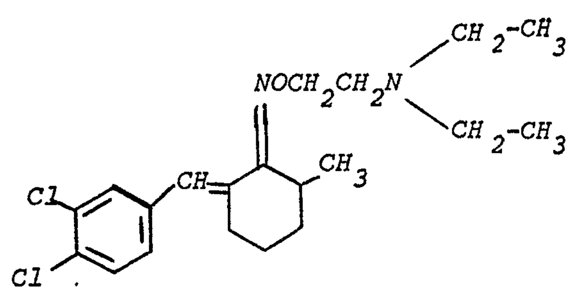 | Chlorhy. 86 | $10^{-6}$ | 60 | | | $10^{-5}$ | 40 |

$C_1$, $C_2$, $C_3$ :concentration en mole/l respectivement en Acetylcholine, chlorure de Baryum et Histamine provoquant une contraction de l'organe.

$DE_{50}$:dose efficace 50 en mg/kg.

REVENDICATIONS

1.- Ethers d'oximes, caractérisés en ce qu'ils répondent à la formule générale (I):

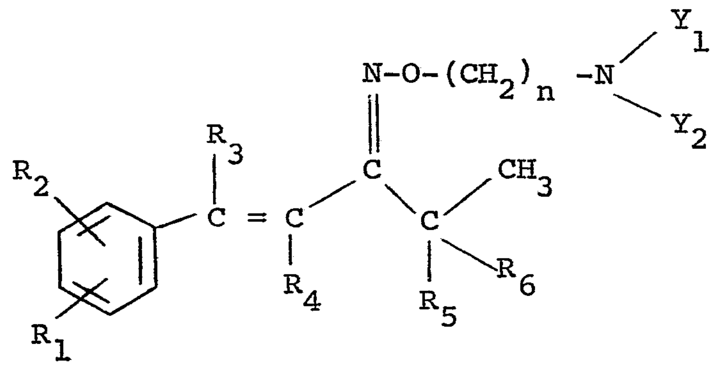

(I)

dans laquelle :

$R_1$ et $R_2$ identiques ou différents représentent un atome d'hydrogène, un atome d'halogène, un radical alkyle de 1 à 4 atomes de carbone, ou bien $R_1$ et $R_2$ pris ensemble représentent un groupe méthylènedioxy,

$R_3$ représente un atome d'hydrogène ou un radical phényle éventuellement substitué par un radical alkyle en $C_1$-$C_4$ ou par un atome d'halogène,

$R_4$ représente un atome d'hydrogène,

$R_5$ représente un groupe méthyle ou pris en combinaison avec le radical $R_4$ représente une chaîne alkylène en $C_3$,

$R_6$ représente un atome d'hydrogène ou le radical méthyle,

$Y_1$ et $Y_2$ identiques ou différents représentent un radical alkyle linéaire ou ramifié ayant de 1 à 4 atomes de carbone, ou bien $Y_1$ et $Y_2$ représentent conjointement une chaîne alkylène en $C_4$-$C_7$ pouvant éventuellement contenir un hétéro-atome d'oxygène ou d'azote,

n est un nombre entier compris entre 1 et 4, ainsi que les sels, les dérivés d'ammonium quaternaire, les stéréo-isomères possibles et leur mélange desdits éthers d'oximes.

2.- Ethers d'oximes selon la revendication 1, caractérisés en ce que n est un nombre entier choisi parmi 2 ou 3.

3.- Ether O-(diéthylaminoéthylique) de l'oxime de la (méthylènedioxy-3,4 phényl)-1 diméthyl-4,4 pentène-1 one-3 .

4.- Ether O-(diméthylaminoéthylique) de l'oxime de la (méthylènedioxy-3,4 phényl)-1 diméthyl-4,4 pentène-1 one-3.

5.- Ether O-(diméthylaminoéthylique) de l'oxime de la (méthylènedioxy-3,4 benzylidène)-2 diméthyl-6,6 cyclohexanone-1 .

6.- Ether O-(diéthylaminopropylique) de l'oxime de la (méthylènedioxy-3,4 benzylidène)-2 méthyl-6 cyclohexanone-1

7.- Procédé de préparation d'éthers d'oximes de formule générale (I) selon la revendication 1, caractérisé en ce que l'on fait réagir une cétone de formule générale (II) ci-après:

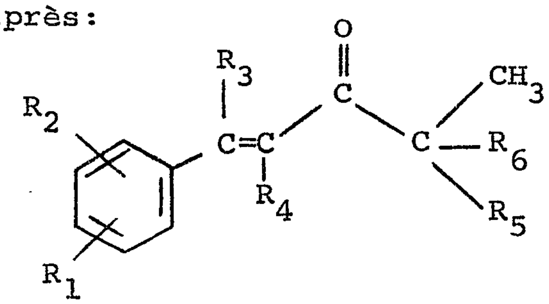

(II)

avec un dérivé d'hydroxylamine de formule générale (III) :

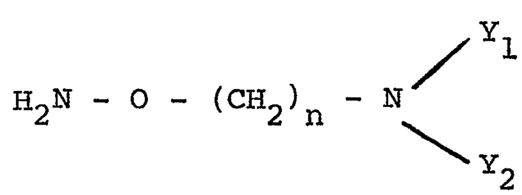

(III)

dans lesquelles $R_1$, $R_2$, $R_3$, $R_4$, $R_5$, $R_6$, $Y_1$, $Y_2$ et n ont la même signification que ci-dessus.

8.- Procédé de préparation d'éthers d'oximes de formule générale (I) selon la revendication 1, caractérisé en ce que l'on fait réagir une oxime de formule générale (IV) ci-après :

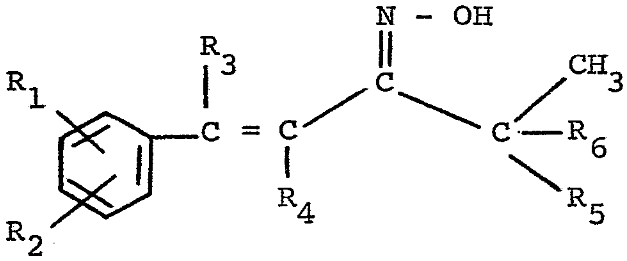

(IV)

avec un dérivé halogéné d'alkylamine de formule générale (V) :

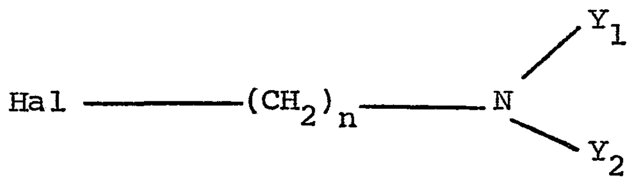

(V)

dans lesquelles $R_1$, $R_2$, $R_3$, $R_4$, $R_5$, $R_6$, $Y_1$, $Y_2$ et n ont la même signification que ci-dessus, dans un solvant inerte en présence d'un agent de condensation basique.

9.- Médicament, caractérisé en ce qu'il comporte comme principe actif une quantité physiologiquement efficace d'au moins un composé selon l'une quelconque des revendications 1 à 6.

10.- Composition pharmaceutique comportant un médicament selon la revendication 9, associé à un véhicule pharmaceutiquement acceptable.

0082059

Office européen des brevets

# RAPPORT DE RECHERCHE EUROPEENNE

Numéro de la demande

EP 82 40 2223

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| X | FR-A-2 339 397 (EGYT)<br>*En entier*<br>--- | 1,7-10 | C 07 D 317/58<br>C 07 C 131/00<br>A 61 K 31/15 |
| A | CH-A- 535 209 (PHILIPS)<br>*Colonnes 1-4; revendications*<br>--- | 1,7-10 | |
| A | CH-A- 535 210 (PHILIPS)<br>*Colonnes 1-4; revendications*<br>----- | 1,7-10 | |
| | | | **DOMAINES TECHNIQUES RECHERCHES (Int. Cl. 3)** |
| | | | C 07 D 317/00<br>C 07 C 131/00 |

Le présent rapport de recherche a été établi pour toutes les revendications

| Lieu de la recherche | Date d'achèvement de la recherche | Examinateur |
|---|---|---|
| LA HAYE | 18-03-1983 | FRANCOIS J.C.L. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons

& : membre de la même famille, document correspondant